# EUROPÄISCHE PATENTSCHRIFT

(11) **EP 4 232 537 B1**
(45) Veröffentlichungstag und Bekanntmachung des Hinweises auf die Patenterteilung: **27.11.2024**
(21) Anmeldenummer: 21793962.8
(22) Anmeldetag: 22.10.2021
(51) Int. Cl.: C11D 1/83, C11D 1/10, C11D 1/72, C11D 3/386, C11D 11/00, C11D 3/00, A61L 2/00, C11D 3/48

(54) **FLÜSSIGES REINIGUNGSMITTELKONZENTRAT, GEBRAUCHSFERTIGE ANWENDUNGSLÖSUNG, DEREN VERWENDUNGEN UND REINIGUNGSVERFAHREN**
LIQUID DETERGENT CONCENTRATE, READY-TO-USE APPLICATION SOLUTION, USES OF SAME AND CLEANING METHODS
CONCENTRÉ DE DÉTERGENT LIQUIDE, SOLUTION D'UTILISATION PRÊTE À L'EMPLOI, SES UTILISATIONS ET PROCÉDÉ DE NETTOYAGE

(30) Priorität: 23.10.2020 EP 20203505
(43) Veröffentlichungstag der Anmeldung: 30.08.2023
(73) Patentinhaber: Chemische Fabrik Dr. Weigert GmbH & Co. KG, 20539 Hamburg (DE)
(72) Erfinder: EISERT, Dennis, 21039 Börnsen (DE); WULFF, Bastian, 22087 Hamburg (DE); SPRINGER, Matthias, 22399 Hamburg (DE)
(74) Vertreter: Glawe, Delfs, Moll
(86) Internationale Anmeldenummer: PCT/EP2021/079352
(87) Internationale Veröffentlichungsnummer: WO 2022/084512

(56) Entgegenhaltungen:
- EP-A1- 1 327 674
- WO-A1-95/33030

## Beschreibung

Die vorliegende Erfindung betrifft ein flüssiges Reinigungsmittelkonzentrat, gebrauchsfertige Anwendungslösungen, deren Verwendungen zur Reinigung und/oder Desinfektion von Gegenständen und Reinigungsverfahren.

Medizinische und chirurgische Instrumente und Apparate werden im Krankenhaus üblicherweise unter Verwendung alkalischer Reinigungsmittel maschinell gereinigt und anschließend chemisch oder thermisch desinfiziert. Stark alkalische Reinigungsmittel können dabei aggressiv gegenüber empfindlichen Oberflächen wirken. Daher werden mildalkalische enzymatische Reinigungsmittel bevorzugt, die jedoch die Nachteile einer nicht zufriedenstellenden Reinigungsleistung sowie hohe Einsatzkonzentrationen aufweisen.

Zudem neigen im Stand der Technik bekannte Reinigungsmittel mit anionischen Tensiden zu einer starken Schaumbildung, welche für die Anwendbarkeit in maschinellen Reinigungsverfahren nachteilig ist. Eine ausgeprägte Schaumbildung führt zu einem Abfall des Dosierpumpendrucks und somit schließlich zum Abbruch des gesamten Reinigungsprozesses. Im Stand der Technik werden daher anionische Tenside unter dem Gesichtspunkt der geringen Schaumbildung ausgewählt und deren Nachteile in puncto Reinigungsleistung in Kauf genommen.

EP 1 327 674 A1 offenbart ein Reinigungsmittelkonzentrat und Verfahren zur Reinigung chirurgischer Instrumente ohne Fettalkoholalkoxylat.

Der Erfindung liegt die Aufgabe zu Grunde, ein flüssiges Reinigungsmittelkonzentrat sowie eine gebrauchsfertige Anwendungslösung hiervon bereitzustellen, die eine sehr gute Reinigungsleistung bei nur einer geringen Einsatzkonzentration ermöglichen und zugleich ein ideales Schaumverhalten sowie eine hohe Materialverträglichkeit auf verschiedenen Werkstoffen aufweisen.

Die Erfindung löst diese Aufgaben durch die Merkmale der Ansprüche 1, 11, 13 und 15. Anspruch 1 umfasst ein flüssiges Reinigungsmittelkonzentrat zur maschinellen Reinigung und/oder Desinfektion von medizinischen und/oder chirurgischen Instrumenten und/oder Instrumenten und/oder Apparaten, umfassend:
a. mindestens ein Fettalkoholalkoxylat,
b. mindestens ein aminosäurebasiertes Tensid,
c. mindestens ein Hydrotrop, und
d. mindestens ein Enzym, vorzugsweise proteolytisches Enzym,
wobei ein pH-Wert des flüssigen Reinigungsmittelkonzentrats 9 oder >9 ist.

Vorzugsweise Ausführungsformen befinden sich in den Unteransprüchen.

Im Rahmen der Erfindung kann das erfindungsgemäße flüssige Reinigungsmittelkonzentrat mit Wasser oder einem wasserhaltigen Lösungsmittelgemisch zu der gebrauchsfertigen Anwendungslösung verdünnt werden. Dieses schließt jedoch nicht aus, dass das flüssige Reinigungsmittelkonzentrat selbst Wasser oder ein wasserhaltiges Lösungsmittelgemisch enthalten kann.

Das flüssige Reinigungsmittelkonzentrat weist vorzugsweise einen pH-Wert 9-12, weiter vorzugsweise 10-12, noch weiter vorzugsweise 10-11, auf.

### 1. Fettalkoholalkoxylate

Das flüssige Reinigungsmittelkonzentrat umfasst mindestens ein Fettalkoholalkoxylat. Vorzugsweise ist das mindestens eine Fettalkoholalkoxylat ausgewählt aus Fettalkoholethoxylaten (FAEO)und Fettalkoholpropoxylaten (FAPO), butylveretherten Fettalkoholethoxylaten (FAEOBV), butylveretherten Fettalkoholpropoxylaten (FAPOBV), methylveretherten Fettalkoholethoxylaten (FAEOMV), methylveretherten Fettalkoholpropoxylaten (FAPOMV), fettalkoholbasierten EO/PO-Copolymeren (FAEOPO), methylverethertem fettalkoholbasierten EO/PO-Copolymeren (FAEOPOMV) und butylverethertem fettalkoholbasierten EO/PO-Copolymeren (FAEOPOBV). Weiter vorzugsweise ist das Fettalkoholalkoxylat ein fettalkoholbasiertes EO/PO-Copolymer.

Das mindestens eine Fettalkoholalkoxylat kann 0-10 EO-Einheiten, vorzugsweise 1-4 EO-Einheiten, weiter vorzugsweise 1-2 EO-Einheiten umfassen. Ferner kann das mindestens eine Fettalkoholalkoxylat 0-8 PO-Einheiten, vorzugsweise 1-8 PO-Einheiten, weiter vorzugsweise 4-8 PO-Einheiten umfassen.

Das Fettalkoholalkoxylat kann mindestens einen C6-C16-Fettal-koholrest, weiter vorzugsweise C12-C15-Fettalkoholrest aufweisen.

Das Fettalkoholalkoxylat ist vorzugsweise ausgewählt aus der Gruppe bestehend aus C12-C15-Fettakoholrest mit 2EO/6PO-Einheiten, C12-C15-Fettalkoholrest mit 8EO/4PO-Einheiten, butyl- oder methylverethertem C12-C14-Fettalkoholrest mit 10EO-Einheiten, C10-C12-Fettalkoholrest mit 6EO/8PO-Einheiten, C12-C14-Fettalkoholrest mit 2EO/4PO-Einheiten, C12-C14-Fettalko-holrest mit 4EO/5PO-Einheiten, C13-C15-Fettalkoholrest mit 5EO/3PO-Einheiten und methylverethertem C13-C15-Fettalkohol-rest mit 5EO/3PO-Einheiten, weiter vorzugsweise ist das Fettalkoholalkoxylat ausgewählt aus C12-C15-Fettakoholrest mit 2EO/6PO-Einheiten, C12-C14-Fettalkoholrest mit 2EO/4PO-Einheiten und C12-C14-Fettalkoholrest mit 4EO/5PO-Einheiten.

Vorzugsweise ist das Fettalkoholalkoxylat in dem flüssigen Reinigungskonzentrat in einem Gewichtsanteil von 0,1 bis 9 Gew.-%, weiter vorzugsweise von 0,4 bis 2 Gew.-%, bezogen auf die Gesamtmasse des flüssigen Reinigungsmittelkonzentrats, vorhanden.

### 2. Aminosäurebasierte Tenside

Das flüssige Reinigungsmittelkonzentrat umfasst mindestens ein aminosäurebasiertes Tensid. Das aminosäurebasierte Tensid ist vorzugsweise ausgewählt aus Verbindungen mit einem gesättigten oder einfach ungesättigten C10-C18-Kohlenstoffrest, vorzugsweise einem gesättigten C12-C16-Kohlenstoffrest.

Das aminosäurebasierte Tensid ist vorzugsweise ausgewählt aus Sarcosinen, Taurinen, Glutaminsäuren und deren Salzen. Bei den Salzen kann es sich um Alkalisalze, vorzugsweise Natrium- und Kaliumsalze handeln. Weiter vorzugsweise handelt es sich bei dem aminosäurebasierten Tensid um ein Sarcosin und dessen Natriumsalz.

Bevorzugte Ausführungsformen des aminosäurebasierten Tensids sind ausgewählt aus Lauroylsarcosin, Oleoylsarcosin, Myristoylsarcosin, Stearoylsarcosin und Lauroylglutaminsäure und deren Natriumsalzen. Besonders bevorzugt sind Lauroylsarcosin und Lauroylglutaminsäure und deren Natriumsalze.

Vorzugsweise ist das aminosäurebasierte Tensid in dem flüssigen Reinigungsmittelkonzentrat in einem Gewichtsanteil von 0,05 bis 5 Gew.-%, weiter vorzugsweise 0,08 bis 2 Gew.-%, noch weiter vorzugsweise von 0,1 bis 2 Gew.-%, bezogen auf die Gesamtmasse des flüssigen Reinigungsmittelkonzentrats, vorhanden.

### 3. Hydrotrope

Das flüssige Reinigungsmittelkonzentrat umfasst mindestens ein Hydrotrop. Im Rahmen der Erfindung handelt es sich bei "Hydrotropen" um Verbindungen, die als Lösungsvermittler fungieren. Erfindungsgemäß handelt es sich hierbei insbesondere um amphiphile Verbindungen mit einem relativ kleinen polaren Teil und einem größeren nicht-polaren Teil, die sowohl in nicht-polaren als auch in polaren Lösungsmitteln löslich sind. Im Vergleich zu Tensiden weisen die Hydrotrope weniger hydrophobe Eigenschaften und eine höhere Löslichkeit in Wasser auf. Der polare Teil stellt eine höhere Löslichkeit in Wasser sicher, während der nicht-polare Teil als funktionelle Gruppe fungiert. Erfindungsgemäße Hydrotrope ermöglichen insbesondere die Formulierung eines klaren und stabilen flüssigen Reinigungsmittelkonzentrats sowie einer klaren gebrauchsfertigen Anwendungslösung. Erfindungsgemäß stellen die als aminosäurebasierte Tenside und/oder Fettalkoholalkoxylate definierten Verbindungen vorzugsweise keine Hydrotrope dar.

Das mindestens eine Hydrotrop ist ausgewählt aus:
- Alkylsulfaten, vorzugsweise C6-C10-Alkylsulfaten und deren Natriumsalze, weiter vorzugsweise Natrium-Octylsulfat und Natrium-Ethylhexylsulfat;
- Alkylsulfonaten, vorzugsweise C6-C10-Alkylsulfonaten;
- aromatischen Sulfonaten, vorzugsweise Xylolsulfonat, p-Toluolsulfonat und deren Natriumsalze;
- Propionaten, vorzugsweise iso-Octyliminodipropionat, n-Octyliminodipropionat, Capryl- und Caprinamphopropionat;
- C4-C10-Ethercarbonsäuren mit 4-10 EO-Einheiten, vorzugsweise Alkyl(8)polyethercarbonsäure mit 8 EO-Einheiten und Alkyl(4-8)polyethercarbonsäure mit 5 EO-Einheiten;
- Alkylglycosiden, Alkyldiglycosiden, Alkylpolyglycosiden und Gemischen hiervon, wobei der Alkylrest vorzugsweise ein verzweigter oder unverzweigter C4-C16-Alkylrest ist und der Glycosidrest vorzugsweise ausgewählt ist aus Hexoseeinheit und Pentoseeinheit, weiter vorzugsweise ausgewählt ist aus Glucopyranoseeinheit und Xylopyranoseinheit.

Besonders bevorzugt sind als Hydrotrop Natrium-Octylsulfat, Natrium-Ethylhexylsulfat oder ein Gemisch hiervon.

Vorzugsweise ist das Hydrotrop in dem flüssigen Reinigungsmittelkonzentrat in einem Gewichtsanteil von 0,05 bis 13 Gew.-%, weiter vorzugsweise von 0,1 bis 7 Gew.-%, noch weiter vorzugsweise 0,15 bis 3,5 Gew.-%,bezogen auf die Gesamtmasse des flüssigen Reinigungsmittelkonzentrats, vorhanden.

### 4. Enzym

Vorzugsweise handelt es sich bei dem Enzym um ein proteolytisches Enzym oder Enzymgemisch.

Vorzugsweise ist das Enzym oder das Enzymgemisch in dem flüssigen Reinigungsmittelkonzentrat in einem Gewichtsanteil von 0,05 bis 4 Gew.-%, weiter vorzugsweise von 0,1 bis 2 Gew.-%, bezogen auf die Gesamtmasse des flüssigen Reinigungsmittelkonzentrats, vorhanden. Die Enzymaktivität beträgt vorzugsweise 30x10⁻² bis 100×10⁻² KNPU/g, weiter vorzugsweise 70×10⁻² bis 85x10⁻² KNPU/g.

### 5. Weitere Bestandteile

Das flüssige Reinigungsmittelkonzentrat kann ferner weitere Bestandteile ausgewählt aus Alkanolaminen, Alkalimetallhydroxiden, Komplexbildnern, Lösungsmitteln, Korrosionsschutzmitteln, Duftstoffen und Farbstoffen umfassen.

Das Alkanolamin ist vorzugsweise ausgewählt aus Monoethanolamin, Triethanolamin, Monoisopropanolamin und Gemischen hiervon. Das Alkanolamin oder dessen Gemisch kann insbesondere dazu dienen die Alkalität des flüssigen Reinigungsmittelkonzentrats einzustellen. Monoethanolamin weist den Vorteil auf, ein guter Proteinreiniger zu sein. Vorzugsweise ist das Alkanolamin oder dessen Gemisch in dem flüssigen Reinigungsmittelkonzentrat in einem Gewichtsanteil von 1 bis 26 Gew.-%, weiter vorzugsweise von 4 bis 18 Gew.-%, bezogen auf die Gesamtmasse des flüssigen Reinigungsmittelkonzentrats, vorhanden.

Vorzugsweise handelt es sich bei dem Alkalimetallhydroxid um Natriumhydroxid und/oder Kaliumhydroxid, weiter vorzugsweise um Kaliumhydroxid. Das Alkalimetallhydroxid dient im Rahmen der Erfindung insbesondere dazu, die Alkalität des flüssigen Reinigungsmittelkonzentrats einzustellen. Vorzugsweise ist Kaliumhydroxid in dem flüssigen Reinigungsmittelkonzentrat in einem Gewichtsanteil von 1 bis 8 Gew.-%, weiter vorzugsweise 2 bis 5 Gew.-%, bezogen auf die Gesamtmasse des flüssigen Reinigungsmittelkonzentrats, vorhanden. Es wurde gefunden, dass die Schädigung von eloxiertem Aluminium trotz dieses hohen Gewichtsanteils von 2 bis 5 Gew.-% an Kaliumhydroxid nicht signifikant gesteigert wird.

Der Komplexbildner kann ausgewählt sein aus der Gruppe bestehend aus Phosphonaten, vorzugsweise aus Salzen der Phosphonobutantricarbonsäure (PBTC), der Aminotrismethylenphosphonsäure (ATMP), der 1-Hydroxyethan-1-1-diphosphonsäure (HEDP), der Diethylentriamin-penta(methylenphosphonsäure) (DTPMP) und Gemische hiervon, weiter vorzugsweise aus den Natriumsalzen der Phosphonobutantricarbonsäure, der Aminotrismethylenphosphonsäure und Gemische hiervon; Aminopolycarbonsäuren, vorzugsweise aus Hydroxyethylethylendiamintriessigsäure (HEDTA), Ethylendiamintetraessigsäure (EDTA), Glutaminsäure-N,N-dies-sigsäure (GLDA), Iminodibernsteinsäure (IDS), Methylglycindiessigsäure (MGDA), Ethylendiamindibernsteinsäure (EDDS), Polyasparaginsäuren, Nitrilotriessigsäure (NTA), Nitrilomonoessigdipropion-säure, Nitrilotripropionsäure, β-Alanindiessigsäure (β-ADA), Diethylentriaminpentaessigsäure, 1,3-Propylendiamintetraessigsäure, 1,2-Propylendiamintetraessigsäure, N-(Alkyl)-ethylendiamintriessigsäure, Ethylendiamintriessigsäure, Cyclohexylen-1,2-diamintetraessigsäure, Serindiessigsäure, Isoserindiessigsäure, L-Asparagindiessigsäure, deren Salzen und Gemischen hiervon, weiter vorzugsweise aus den Natriumsalzen der HEDTA, der MGDA und Gemischen hiervon; und Hydroxycarbonsäuren, Hydroxypolycarbonsäuren und deren Salzen, vorzugsweise Gluconsäure, Glucoheptansäure, Äpfelsäure, Weinsäure, Schleimsäure, Milchsäure, Glutarsäure, Zitronensäure, Tartronsäure, Lactobionsäure und Saccharosemono-, -di- und -tricarbonsäure und deren Salzen, weiter vorzugsweise aus dem Natriumsalz der Glucoheptonsäure. Weiter vorzugsweise handelt es sich bei dem Komplexbildner um ein Gemisch aus mindestens einem Phosphonat und mindestens einer Aminopolycarbonsäure, Hydroxycarbonsäure oder deren Salz. Das Komplexbildnergemisch kann ferner eine weitere Aminopolycarbonsäure umfassen, die ebenfalls ausgewählt ist aus den zuvor definierten Aminopolycarbonsäuren und deren Salzen, wobei die erste und die zweite Aminopolycarbonsäure verschieden voneinander sind. Das Phosphonat oder das Phosphonatgemisch können in dem flüssigen Reinigungsmittelkonzentrat in einem Gewichtsanteil von 1 bis 13 Gew.-%, vorzugsweise von 2 bis 10 Gew.-%, weiter vorzugsweise 3 bis 8 Gew.-%, bezogen auf die Gesamtmasse des flüssigen Reinigungsmittelkonzentrats, vorhanden sein. Die Aminopolycarbonsäuren, die Hydroxycarbonsäuren, die Hydroxypolycarbonsäure, deren Salze oder deren Gemische können in dem flüssigen Reinigungsmittelkonzentrat in einem Gewichtsanteil von 1 bis 10 Gew.-%, vorzugsweise von 2 bis 8 Gew.-%, weiter vorzugsweise von 3 bis 6 Gew.-%,bezogen auf die Gesamtmasse des flüssigen Reinigungsmittelkonzentrats, vorhanden sein.

Bei dem Lösungsmittel kann es sich um Wasser oder ein wasserhaltiges Lösungsmittelgemisch handeln. Bevorzugt sind Lösungsmittelgemische die neben Wasser organische Lösungsmittel ausgewählt aus Ethanol, 2-Propanol, Glykolen, Glycerin und Gemischen hiervon umfassen. Ein bevorzugtes Glykol ist 1,2-Propylenglykol. Vorzugsweise ist das organische Lösungsmittel in dem flüssigen Reinigungsmittelkonzentrat in einem Gewichtsanteil von 0,5 bis 10 Gew.-%, weiter vorzugsweise von 3 bis 7 Gew.-%, bezogen auf die Gesamtmasse des flüssigen Reinigungsmittelkonzentrats, vorhanden.

Vorzugsweise ist Wasser in dem flüssigen Reinigungsmittelkonzentrat in einem Gewichtsanteil von 30 bis 90 Gew.-%, weiter vorzugsweise von 35 bis 70 Gew.-%, noch weiter vorzugsweise von 35 bis 60 Gew.-%, noch weiter vorzugsweise 35 bis 50 Gew.-%, noch weiter vorzugsweise 35 bis 45 Gew.-%, bezogen auf die Gesamtmasse des flüssigen Reinigungsmittelkonzentrats, vorhanden.

Das flüssige Reinigungsmittelkonzentrat kann zudem Phosphorsäure enthalten, welche vorzugsweise als Korrosionsschutzmittel dient. Vorzugsweise ist Phosphorsäure in dem flüssigen Reinigungsmittelkonzentrat in einem Gewichtsanteil von 0,1 bis 20 Gew.-%, weiter vorzugsweise 0,2 bis 7 Gew.-%, noch weiter vorzugsweise 0,3 bis 5 Gew.-%,bezogen auf die Gesamtmasse des flüssigen Reinigungsmittelkonzentrats, vorhanden.

Eine Obergrenze für einen Gesamtgewichtsanteil an Fettalkoxylakoholat, aminosäurebasiertem Tensid und Hydrotrop in dem flüssigen Reinigungsmittelkonzentrat beträgt vorzugsweise 30 Gew.-%, weiter vorzugsweise 20 Gew.-%, noch weiter vorzugsweise 13 Gew.-%, noch weiter vorzugsweise 10 Gew.-%, noch weiter vorzugsweise 8 Gew.-%, noch weiter vorzugsweise 5 Gew.-%, bezogen auf die Gesamtmasse des flüssigen Reinigungsmittelkonzentrats.

Vorzugsweise sind Tenside (d.h. kationische, anionische, zwitterionische, nichtionische Tenside) in dem flüssigen Reinigungsmittelkonzentrat höchstens in einem Gewichtsanteil von bis zu 30 Gew.-%, weiter vorzugsweise bis zu 20 Gew.-%, noch weiter vorzugsweise bis zu 18 Gew.-%, noch weiter vorzugsweise bis zu 13 Gew.-%, noch weiter vorzugsweise bis zu 9 Gew.-%, noch weiter vorzugsweise bis zu 7,5 Gew.-%, bezogen auf die Gesamtmasse des flüssigen Reinigungsmittelkonzentrats, vorhanden.

Anionische Tenside sind in dem flüssigen Reinigungsmittelkonzentrat vorzugsweise höchstens in einem Gewichtsanteil von bis zu 20 Gew.-%, weiter vorzugsweise bis zu 18 Gew.-%, noch weiter vorzugsweise bis zu 13 Gew.-%, noch weiter vorzugsweise bis zu 8 Gew.-%, noch weiter vorzugsweise bis zu 5 Gew.-%, noch weiter vorzugsweise bis zu 3 Gew.-%, bezogen auf die Gesamtmasse des flüssigen Reinigungsmittelkonzentrats, vorhanden.Die Erfindung beruht auf der überraschenden Erkenntnis, dass die Kombination von Fettalkoholalkoxylat, aminosäurebasiertem Tensid und Hydrotrop in dem flüssigen Reinigungsmittelkonzentrat die folgenden technischen Wirkungen/Vorteile aufweist. Das Konzentrat, enthaltend eben diese drei Bestandteile, stellt eine schaumarme Reinigungsformulierung dar, die eine sehr gute Reinigungsleistung bei nur einer geringen Einsatzkonzentration erzielt und zugleich eine hohe Materialverträglichkeit bei der Anwendung auf verschiedenen Werkstoffen aufweist.

Die in dem erfindungsgemäßen flüssigen Reinigungsmittelkonzentrat enthaltenen Wirkstoffe können in einer deutlich geringeren Dosierung als bei anderen im Stand der Technik bekannten Reinigungsmittel eingesetzt werden. Dieses ist insbesondere auf einen synergistischen Effekt hinsichtlich der Reinigungsleistung zurückzuführen. Die erzielte Reinigungsleistung des flüssigen Reinigungsmittelkonzentrats enthaltend die Kombination von Fettalkoholalkoxylat, aminosäurebasiertem Tensid und Hydrotrop ist deutlich besser als die, der jeweiligen Einzelkomponenten.

Anionische und auch aminosäurebasierte Tenside sind in der Regel starke Schaumbildner und werden im Stand der Technik oftmals gezielt eingesetzt, um diese Eigenschaft bei Reinigungsformulierungen zu verstärken. Im Rahmen der Erfindung ist ein starkes Schäumen jedoch gerade nicht gewünscht. Für die Anwendung in maschinellen Reinigungsverfahren, beispielsweise mittels Instrumentenspülmaschine oder Reinigung- und Desinfektionsgeräten (RDG), sind schaumarme Bestandteile erforderlich. Denn eine ausgeprägte Schaumbildung führt bei der maschinellen Reinigung zu einem Abfall des Dosierpumpendrucks und somit schließlich zu einem Abbruch des Reinigungsprozesses. Überraschenderweise wird die Schaumbildung durch die Kombination von Fettalkoholalkoxylat, aminosäurebasiertem Tensid und Hydrotrop unterdrückt. Insbesondere wird die schaumbildende Wirkung des aminosäurebasierten Tensids durch die Zugabe des Fettalkoholalkoxylats gedämpft, so dass ein gewünschtes Schaumverhalten des flüssigen Reinigungsmittelkonzentrats eingestellt kann werden.

Im Stand der Technik erfolgt das Eindosieren von tensidhaltigen, enzymatischen, mildalkalischen, flüssigen Reinigungsmitteln bei der maschinellen Reinigung üblicherweise bei einer Wassertemperatur von etwa 40°C. Dieses ist notwendig, da bei geringeren Temperaturen eine zu starke Schaumneigung der Reiniger vorliegt. Nachteilig hieran ist allerdings, dass die Laufzeit der Reinigungsprogramme länger ist, da zunächst die Aufheizzeit von der Einlauftemperatur (meist ca. 18-22°C) bis auf 40°C abgewartet werden muss, bevor der Reiniger wirken kann. Das erfindungsgemäße flüssige Reinigungsmittelkonzentrat ermöglicht es hingegen eine Kaltdosierung bereits direkt nach dem Wassereinlauf, bei einer Temperatur vorzugsweise von 38°C oder weniger, weiter vorzugsweise von 18 bis 35°C, noch weiter vorzugsweise von 20 bis 30°C, noch weiter vorzugsweise von 22 bis 27°C, noch weiter vorzugsweise bei etwa 25°C, durchzuführen, ohne dass es zu einem Programmabbruch aufgrund von zu starker Schaumentwicklung kommt. Eine solche Kaltdosierung ist mit den aus dem Stand der Technik bekannten und marktüblichen flüssigen Reinigungsmitteln derzeit nicht möglich. Das Kaltdosieren des flüssigen Reinigungsmittelkonzentrats weist eigenständig erfinderischen Gehalt auf.

Eine weitere wichtige Anforderung an flüssige Reinigungsmittel ist eine möglichst hohe Materialverträglichkeit bei der Anwendung auf verschiedenen Werkstoffen. Insbesondere im Hinblick auf medizinische und/oder chirurgische Instrumente und/oder Apparate ist der Korrosionsschutz von Edelstahl- und (farb-)eloxierten Aluminiumteilen von Bedeutung. Überraschenderweise weist das erfindungsgemäße flüssige Reinigungsmittelkonzentrat eine hohe Materialverträglichkeit auf. Insbesondere die Anwesenheit des aminosäurebasierten Tensids führt zu einem deutlich verbesserten Korrosionsinhibitionsverhalten auf Edelstahl- und (farb-)eloxierten Aluminiumteilen. Zudem wird überraschenderweise bei einer häufigeren Anwendung des erfindungsgemäßen flüssigen Reinigungsmittelkonzentrats ein verbesserter Glanz sowie eine verbesserte Haptik insbesondere von Edelstahlteilen beobachtet.

Im Rahmen der Erfindung fungiert das Hydrotrop ferner als Lösungsvermittler für das Fettalkoholalkoxylat und zur Klarstellung der Formulierung innerhalb eines bestimmten Temperaturbereichs. Als Untersuchungsparameter können hierfür der Trübungspunkt der flüssigen Reinigungsformulierungen sowie der Dosierpumpendruck während eines maschinellen Spülvorgangs herangezogen werden. Überraschenderweise wird durch die erfindungsgemäße Kombination der Bestandteile ein Trübungspunkt >40°C erreicht. Dieses ist besonders vorteilhaft, da dieses in der Regel auch die Anwendungstemperatur bzw. die Lagertemperatur im RDG ist. Der Trübungspunkt ohne die erfindungsgemäße Kombination läge hingegen bei nur ca. 20°C.

Gegenstand der Erfindung ist ferner eine gebrauchsfertige Anwendungslösung umfassend 0,05 bis 99,9% des erfindungsgemäßen flüssigen Reinigungsmittelkonzentrats, wobei ein pH-Wert der gebrauchsfertigen Anwendungslösung 9 oder >9, vorzugsweise 9-12, weiter vorzugsweise 10-12, noch weiter vorzugsweise 10-11, ist.

Vorzugsweise umfasst die gebrauchsfertige Anwendungslösung 0,05 bis 10%, weiter vorzugsweise 0,1 bis 1% des erfindungsgemäßen flüssigen Reinigungsmittelkonzentrats.

Die zuvor aufgeführten Bestandteile, Eigenschaften und vorteilhaften Effekte der gebrauchsfertigen Anwendungslösung entsprechen dabei denen, die zuvor für das flüssige Reinigungsmittelkonzentrat definiert wurden. Allerdings sind die Bestandteile in der gebrauchsfertigen Anwendungslösung in den folgenden Gewichtsanteilen vorhanden:
Vorzugsweise ist das Fettalkoholalkoxylat in der gebrauchsfertigen Anwendungslösung in einem Gewichtsanteil von 0,00005 bis 0,9 Gew.-%, weiter vorzugsweise 0,0004 bis 0,02 Gew.-%, bezogen auf die Gesamtmasse der gebrauchsfertigen Anwendungslösung, vorhanden.

Vorzugsweise ist das aminosäurebasierte Tensid in der gebrauchsfertigen Anwendungslösung in einem Gewichtsanteil von 0,000025 bis 0,5 Gew.-%, weiter vorzugsweise 0,0001 bis 0,02 Gew.-%, bezogen auf die Gesamtmasse der gebrauchsfertigen Anwendungslösung, vorhanden.

Vorzugsweise ist das Hydrotrop in der gebrauchsfertigen Anwendungslösung in einem Gewichtsanteil von 0,000025 bis 1,3 Gew.-%, weiter vorzugsweise von 0,0001 bis 0,07 Gew.-%, noch weiter vorzugsweise von 0,00015 bis 0,035 Gew.-% bezogen auf die Gesamtmasse der gebrauchsfertigen Anwendungslösung, vorhanden.

Vorzugsweise ist das Enzym oder Enzymgemisch in der gebrauchsfertigen Anwendungslösung in einem Gewichtsanteil von 0,000025 bis 0,4 Gew.-%, weiter vorzugsweise von 0,0001 bis 0,02 Gew.-%, bezogen auf die Gesamtmasse der gebrauchsfertigen Anwendungslösung, vorhanden.

Das Alkanolamin oder dessen Gemisch kann in der gebrauchsfertigen Anwendungslösung in einem Gewichtsanteil von 0,0005 bis 2,6 Gew.-%, vorzugsweise von 0,004 bis 0,18 Gew.-%, bezogen auf die Gesamtmasse der gebrauchsfertigen Anwendungslösung, vorhanden sein.

Der Komplexbildner oder das Komplexbildnergemisch kann in der gebrauchsfertigen Anwendungslösung in einem Gewichtsanteil von 0,001 bis 2,3 Gew.-%, vorzugsweise 0,004 bis 0,18 Gew.-%, weiter vorzugsweise 0,006 bis 0,14 Gew.-%, bezogen auf die Gesamtmasse der gebrauchsfertigen Anwendungslösung, vorhanden sein.

Das organische Lösungsmittel kann in der gebrauchsfertigen Anwendungslösung in einem Gewichtsanteil von 0,00025 bis 1,0 Gew.-%, vorzugsweise 0,003 bis 0,07 Gew.-%, bezogen auf die Gesamtmasse der gebrauchsfertigen Anwendungslösung, vorhanden sein.

Wasser kann in der gebrauchsfertigen Anwendungslösung in einem Gewichtsanteil von 90,0 bis 99,985 Gew.-%, vorzugsweise 95,0 bis 99,98 Gew.-%, weiter vorzugsweise 99,6 bis 99,96 Gew.-%, bezogen auf die Gesamtmasse der gebrauchsfertigen Anwendungslösung, vorhanden sein.

Phosphorsäure kann in der gebrauchsfertigen Anwendungslösung in einem Gewichtsanteil von 0,00005 bis 2,5 Gew.-%, weiter vorzugsweise 0,0002 bis 0,14 Gew.-%, noch weiter vorzugsweise 0,0003 bis 0,07 Gew.-%, bezogen auf die Gesamtmasse der gebrauchsfertigen Anwendungslösung, vorhanden sein.

Eine Obergrenze für einen Gesamtgewichtsanteil an Fettalkoxylakoholat, aminosäurebasiertem Tensid und Hydrotrop in der gebrauchsfertigen Anwendungslösung beträgt vorzugsweise 2,7 Gew.-%, weiter vorzugsweise 1 Gew.-%, noch weiter vorzugsweise 0,4 Gew.-%, noch weiter vorzugsweise 0,1 Gew.-%, noch weiter vorzugsweise 0,075 Gew.-%, bezogen auf die Gesamtmasse der gebrauchsfertigen Anwendungslösung.

Vorzugsweise sind Tenside (d.h. kationische, anionische, zwitterionische, nichtionische Tenside) in der gebrauchsfertigen Anwendungslösung höchstens in einem Gewichtsanteil von bis zu 3 Gew.-%, weiter vorzugsweise bis zu 2,7 Gew.-%, noch weiter vorzugsweise bis zu 1,5 Gew.-%, noch weiter vorzugsweise bis zu 1 Gew.-%, noch weiter vorzugsweise bis zu 0,075 Gew.-%, bezogen auf die Gesamtmasse der gebrauchsfertigen Anwendungslösung, vorhanden.

Anionische Tenside sind in der gebrauchsfertigen Anwendungslösung vorzugsweise höchstens in einem Gewichtsanteil von bis zu 2,8 Gew.-%, weiter vorzugsweise bis zu 2 Gew.-%, noch weiter vorzugsweise bis zu 1,8 Gew.-%, noch weiter vorzugsweise bis zu 1 Gew.-%, noch weiter vorzugsweise bis zu 0,5 Gew.-%, noch weiter vorzugsweise bis zu 0,1 Gew.-%, noch weiter vorzugsweise bis zu 0,055 Gew.-%, bezogen auf die Gesamtmasse der gebrauchsfertigen Anwendungslösung, vorhanden.

Gegenstand der Erfindung ist zudem die Verwendung des erfindungsgemäßen flüssigen Reinigungsmittelkonzentrats oder der erfindungsgemäßen gebrauchsfertigen Anwendungslösung zur Reinigung und/oder Desinfektion von Gegenständen, vorzugsweise zur maschinellen Reinigung und/oder Desinfektion von Gegenständen. In einer vorteilhaften Ausführungsform werden das flüssige Reinigungsmittelkonzentrat oder die gebrauchsfertige Anwendungslösung vorzugsweise kalt dosiert, weiter vorzugsweise bei einer Temperatur von 38°C oder weniger, noch weiter vorzugsweise von 18 bis 35°C, noch weiter vorzugsweise von 20 bis 30°C, noch weiter vorzugsweise von 22 bis 27°C, noch weiter vorzugsweise bei etwa 25°C.

Eine maschinelle Reinigung erfolgt im Rahmen der Erfindung ohne menschliches Zutun während eines automatischen Programmablaufes, vorzugsweise in einer Instrumentenspülmaschine oder im RDG. Mit der Formulierung "Reinigung und/oder Desinfektion" wird erfindungsgemäß zum Ausdruck gebracht, dass das flüssige Reinigungsmittelkonzentrat und die gebrauchsfertige Anwendungslösung sowohl bei der Kombination von Reinigung und Desinfektion in einem einzigen Verfahrensschritt als auch bei Programmabläufen verwendet werden kann, bei denen einem Reinigungsschritt ein separater Desinfektionsschritt nachfolgt. Bei den Gegenständen handelt es sich vorzugsweise um medizinische und/oder chirurgische Instrumente und/oder Apparate.

Gegenstand der Erfindung ist ferner das Verfahren zur Reinigung von medizinischen und/oder chirurgischen Instrumenten und/oder Apparaten, gekennzeichnet durch folgende Schritte:
a) Herstellen einer gebrauchsfertigen Anwendungslösung nach Ansprüchen 11 oder 12,
b) Reinigen der medizinischen und/oder chirurgischen Instrumente und/oder Apparate mit der gebrauchsfertigen Anwendungslösung.

In einer vorteilhaften Ausführungsform wird die gebrauchsfertige Anwendungslösung vorzugsweise kalt hergestellt, weiter vorzugsweise bei einer Temperatur von 38°C oder weniger, noch weiter vorzugsweise von 18 bis 35°C, noch weiter vorzugsweise von 20 bis 30°C, noch weiter vorzugsweise von 22 bis 27°C, noch weiter vorzugsweise bei etwa 25°C. Die Herstellung der gebrauchsfertigen Anwendungslösung kann dabei durch die Dosierung des erfindungsmäßen flüssigen Reinigungskonzentrats erfolgen. Optional kann die gebrauchsfertige Anwendungslösung auch manuell ausgehend von dem erfindungsgemäßen flüssigen Reinigungsmittelkonzentrat hergestellt werden.

Die Erfindung wird nun anhand einiger vorteilhafter Ausführungsformen unter Bezugnahmen auf die beigefügten Zeichnungen beispielhaft beschrieben. Es zeigt:
- Figur 1:: Druck-Zeit-Diagramme eines maschinellen Spülzyklus mit gutem Pumpendruckverhalten (oben) und schlechtem Pumpendruckverhalten (unten)
- Figur 2:: Druck-Zeit-Diagramme eines maschinellen Spülzyklus mit gebrauchsfertigen Anwendungslösungen enthaltend die erfindungsgemäßen Fettalkoholalkoxylate a) C12-C15-Fettakoholrest mit 2EO/6PO-Einheiten, b) C12-C14-Fettalkoholrest mit 2EO/4PO-Einheiten und c) C12-C14-Fettalkoholrest mit 4EO/5PO-Einheiten
- Figur 3:: Korrosionsverhalten auf Grauguss GG25 getestet in Anlehnung an DIN 51360 Teil 2 mit gebrauchsfertigen Anwendungslösungen umfassend a) Natrium-Lauroylsarcosinat, b) Natrium-Oleoylsarcosinat, c) Natrium-Myristoylsarcosinat, d) Natrium-Stearoylsarcosinat, e) Natrium-Lauroylglutamat als erfindungsgemäße aminosäure-basierte Tenside und f) Natrium-Cumolsulfonat als Referenzformulierung
- Figur 4:: Säulendiagramm mit quantitativen Ergebnissen für das Ausmaß der Korrosion mit Graugussspänen GG25
- Figur 5:: quantitative Bestimmung der Blutrückstände nach Tauchversuchen mit Schafsblut und einer Mischung aus Schafsblut und Betaisodona-Tinktur
- Figur 6:: Druck- und Temperaturkurven für die Kaltwasserdosierung eines erfindungsgemäßen flüssigen Reinigungsmittelkonzentrats in einer Konzentration von 3 ml/l bei 25°C
- Figur 7:: Druck- und Temperaturkurven für die Kaltwasserdosierung der aus dem Stand der Technik bekannten und marktüblichen Reiniger mit der jeweils standardmäßig empfohlenen Konzentration bei 25°C

### 1. Fettalkoholalkoxylat

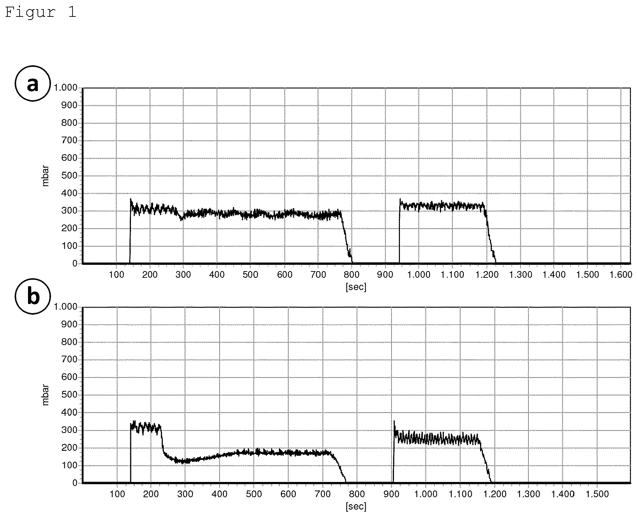

Es wurden sieben verschiedene Fettalkoholalkoxylate mit variabler Kettenlänge des Fettalkoholrestes sowie unterschiedlichem Ethoxylierungs- bzw. Propoxylierungsgrad (d.h. FA C12-C15 mit 2EO/6PO, FA C12-C14 mit 2EO/4PO, FA C12-C14 mit 4EO/5PO, FA C12-C15 mit 8EO/4PO, butylverethertem FA C12-C14 mit 10EO, FA C12-C12 mit 6EO/8PO, methylverethertem FA C13-C15 mit 5EO/3PO) hinsichtlich ihrer schaumdämpfenden Eigenschaften in einer gebrauchsfertigen Anwendungslösung mit einer ansonsten gleichbleibenden Reinigungsformulierung geprüft. Die jeweilige Eignung des Fettalkoholalkoxylats für den Einsatz in einem maschinellen Reinigungsverfahren wurde anhand der Messung des Dosierpumpendrucks während eines gesamten Reinigungsvorgangs beurteilt (vgl. Figur 1).

Figur 1 zeigt einen Vergleich zwischen Druck-Zeit-Diagrammen eines maschinellen Spülzyklus mit einem sehr gutem Pumpendruckverhalten (oben) und einem schlechtem Pumpendruckverhalten (unten).

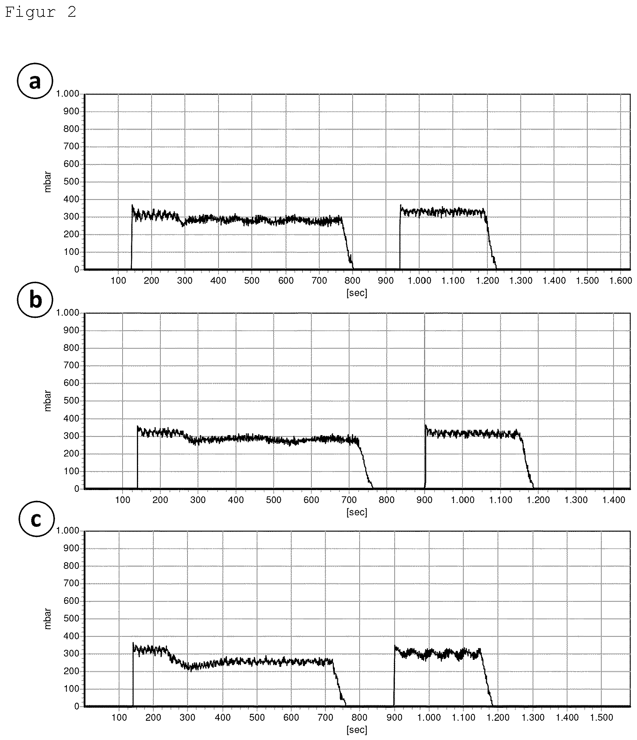

Als besonders vorteilhaft stellten sich die Fettalkoholalkoxylate a) C12-C15-Fettakoholrest mit 2EO/6PO-Einheiten, b) C12-C14-Fettalkoholrest mit 2EO/4PO-Einheiten und c) C12-C14-Fettalkoholrest mit 4EO/5PO-Einheiten heraus. Die zugehörigen Druck-Zeit-Diagramme der erfindungsgemäßen Fettalkoholalkoxylate des jeweiligen maschinellen Spülzyklus sind in Figur 2 gezeigt. Die Fettalkoholalkoxylate weisen allesamt ein sehr gutes Pumpendruckverhalten auf.

### 2. Aminosäurebasiertes Tensid

Es wurden fünf verschiedene aminosäurebasierte Tenside (d.h. Natrium-Lauroylsarcosinat, Natrium-Oleoylsarcosinat, Natrium-Myristoylsarcosinat, Natrium-Stearoylsarcosinat, Natrium-Lauroylglutamat) bezüglich ihrer korrosionsinhibierenden Eigenschaften in einer gebrauchsfertigen Anwendungslösung mit einer ansonsten gleichbleibenden Reinigungsformulierung im Vergleich zu einer Referenzformulierung, die ein gängiges nicht-aminosäurebasiertes Tensid (d.h. Natrium-Cumolsulfonat) enthält, untersucht. Zur Beurteilung wurden Korrosionsversuche mit Graugussspänen GG25 in Anlehnung an DIN 51360 Teil 2 durchgeführt.

### 1. Korrosionsversuche mit Graugussspänen GG25 in Anlehnung an DIN 51360 Teil 2

### a. Geräte und Materialien

- Petrischalen Ø 100 mm (Glas oder Kunststoff)
- Filterpapier Ø 70 mm 589 der Fa. Whatman aschefrei, mittelschnell filtrierend
- Grauguß GG 25-Späne nach DIN 51360 T2 (Fa. Riegger Industriehandel, Artikel 03-39)
- VE-Wasser

### b. Durchführung

Auf das in die Petrischale eingelegte Filterpapier wurden mit Hilfe eines Löffelspatels 2 g ± 0,1 g der Späne eingewogen.

Die Späne wurden möglichst mittig auf einer Fläche von Ø 40-50 mm verteilt. Die Späne und das Filterpapier wurden gleichmäßig mit 2 ml der 2,5%igen gebrauchsfertigen Anwendungslösung benetzt und die Petrischale mit dem Deckel verschlossen. Die so vorbereiteten Proben wurden 2 Stunden ± 10 Minuten bei Raumtemperatur (20-25°C) ohne direkte Sonneneinstrahlung oder Zugluft gelagert. Die Späne wurden entfernt und verworfen. Das Filterpapier wurde unter fließendem VE-Wasser abgespült und für 5-10 Sekunden in Aceton geschwenkt. Das Filterpapier wurde bei Raumtemperatur (20-25°C) getrocknet. Sofort nach der Trocknung wurde der Korrosionsgrad bestimmt. Jede Prüfung wurde als Doppelbestimmung durchgeführt.

### c. Auswertung

Für die Auswertung wurde, anstelle einer visuellen Beurteilung, die Fläche der auftretenden Korrosion zur Gesamtfläche des verwendeten Filterpapiers in Beziehung gesetzt. Die Integrale der Flächen wurden mithilfe der Software ImageJ ermittelt.

### d. Ergebnis

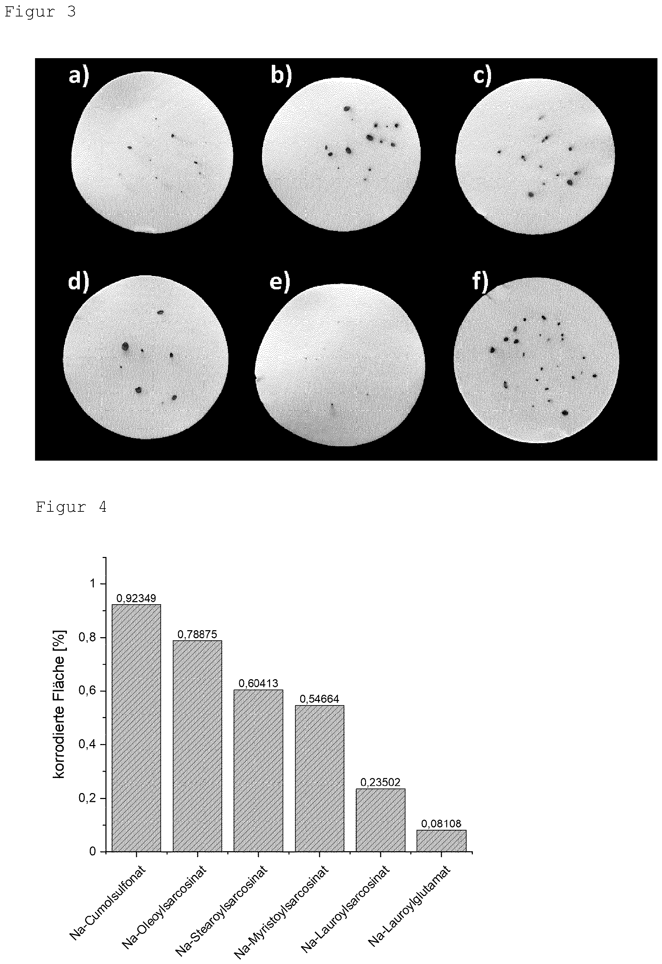

Alle Versuchsansätze der gebrauchsfertigen Anwendungslösungen, die ein aminosäurebasiertes Tensid enthalten, weisen gegenüber der Referenzformulierung ohne aminosäurebasiertes Tensid ein deutlich verbessertes Korrosionsinhibitionsverhalten auf. Die besten Ergebnisse werden mit den gebrauchsfertigen Anwendungslösungen enthaltend die aminosäurebasierten Tenside Natrium-Lauroylsarcosinat und Natrium-Lauroylglutamat erzielt. Dabei ist das Korrosionsverhalten auf Grauguss GG25 aus Figur 3 ersichtlich. Das Ausmaß der Korrosion in den Versuchen mit Graugussspänen konnte durch anschließende Integration der korrodierten Flächen quantitativ ermittelt werden (siehe Figur 4).

### 3. Hydrotrop

Es wurden acht verschiedene Verbindungen unterschiedlicher Stoffklassen, d.h. jeweils ausgewählt aus Alkylethercarbonsäuren, Alkylsulfaten, Alkylsulfonaten, aromatischen Sulfonaten, Alkyl-, Alkyldi- und Alkylpolyglycosiden, auf ihre Eignung als Hydrotrop in gebrauchsfertigen Anwendungslösungen getestet. Als Untersuchungsparameter wurden der Trübungspunkt sowie der Dosierpumpendruck während eines maschinellen Spülvorgangs zur Beurteilung herangezogen.

Überraschenderweise wird durch die erfindungsgemäße Kombination der Bestandteile ein Trübungspunkt >40°C für die gebrauchsfertigen Anwendungslösungen erreicht. Dieses ist besonders vorteilhaft, da dieses in der Regel auch die Anwendungstemperatur bzw. die Lagertemperatur im RDG ist. Insbesondere die Anwendungslösungen umfassend Alkylsulfate als Hydrotrop zeigen sehr gute Ergebnisse. Der Trübungspunkt ohne die erfindungsgemäße Kombination liegt hingegen bei nur ca. 20°C.

### 4. Kombination Fettalkoholalkoxylat, aminosäurebasiertes Tensid und Hydrotrop

Verschiedene Kombinationen von aminosäurebasierten Tensiden und Hydrotropen mit dem besten Fettalkoholalkoxylat aus dem obigen Abschnitt wurden in gebrauchsfertigen Anwendungslösungen untersucht.

In Tauchbadversuchen wurden die Rezepturvarianten hinsichtlich ihrer Reinigungsleistung bezüglich Schafsblut und einer Mischung aus Blut und Betaisodona-Tinktur geprüft. In maschinellen Spülversuchen wurde der Dosierpumpendruck als Maß für das Schaumverhalten im Verlauf eines vollständigen Reinigungszyklus verfolgt. Dabei wurden zusätzlich 10 ml Blut in die Reinigungsflotte gegeben, wodurch es zu einer verstärkten Schaumbildung kommt und die Aufbereitung stark verschmutzten Instrumentariums simuliert werden kann.

### 1. Reinigungsversuche im Tauchbad

### a. Geräte und Materialien

- Edelstahlplättchen (leicht angeraut, Fläche 1 cm x 9 cm)
- Schafblut heparinisiert mit 10 IE/ml
   Protaminsulfat oder Protaminchlorid: ACILA GmbH
- Betaisodona (10% Povidon Iodlösung)
- Markierungspunkte Ø 8mm versch. Farben
- VE-Wasser

### b. Durchführung

Herstellung der Prüfplättchen heparinisiertes, reaktiviertes Schafblut:
Das heparinisierte Schafblut und das Protaminsulfat/Protaminchlorid wurden bis zum Versuch im Klimaschrank bei 6°C gelagert. Für die Herstellung der Prüfanschmutzung sollte das Schafblut und das Protaminsulfat/Protaminchlorid eine Temperatur von 20°C erreicht haben. Die fettfreien Edelstahlplättchen wurden auf ein Rack gespannt und sollten horizontal möglichst gerade ausgerichtet sein.

75 µl Protaminsulfat oder Protaminchlorid wurden mit 5 ml heparinisierten Schafblut in einem 50 ml-Becherglas auf einem Magnetrührer kurz gemischt. Von dieser Lösung wurden auf jedes Plättchen 100 µl pipettiert und mit einer Impföse gleichmäßig verteilt ohne die Befestigungslöcher und die seitlichen Flächen dabei zu kontaminieren. Danach erfolgte eine Inkubation jeder Charge für 1 Stunde bei Raumtemperatur in wasserdampfgesättigter Luft (100 % Luftfeuchtigkeit LF). Das Gestell der Plättchen kann ins VE-Wasser eintauchen, jedoch müssen die Plättchen oberhalb des Wasserspiegels gelagert werden. Zur Einstellung von 100% LF wurde der Boden einer 8,5 1 Plastikdose mit mindestens 1 l VE-Wasser gefüllt. Das VE-Wasser muss den Boden der horizontal aufgestellten Schale vollständig bedecken. Die Schale wurde mind. 2 Stunden vor Start mit einem Deckel versehen (Konditionierung der Atmosphäre). Nach 1 Stunde wurden die nassen Prüfkörper mit der koagulierten Blutanschmutzung aus der Plastikschale genommen und bei Raumtemperatur getrocknet.

Die Qualität der trockenen Prüfplättchen wurde überprüft. Plättchen mit Luftblasen auf der Anschmutzung oder die Unregelmäßigkeiten aufweisen wurden aussortiert. Auf alle anderen Plättchen wurde je ein grüner Markierungspunkt geklebt. Bis zur Verwendung im Tauchversuch wurden die Prüfplättchen in Reagenzgläsern mit Schraubverschluss bei Raumtemperatur gelagert.

### Herstellung Prüfplättchen Iodblut:

Das defibrinierte Schafblut wurde bis zum Versuch im Klimaschrank bei 6°C gelagert. Für die Herstellung der Prüfanschmutzung sollte das Schafblut eine Temperatur von 20°C erreicht haben. Die fettfreien Edelstahlplättchen wurden auf ein Rack gespannt und sollten horizontal möglichst gerade ausgerichtet sein.

Das defibrinierte Schafblut wurde im Verhältnis 1:1 mit Betaisodona in einem 50 ml-Becherglas auf einem Magnetrührer kurz gemischt. Von dieser Lösung wurden auf jedes Plättchen 200 µl pipettiert und mit einer Impföse gleichmäßig verteilt ohne die Befestigungslöcher und die seitlichen Flächen dabei zu kontaminieren. Die Prüfplättchen trockneten bei Raumtemperatur etwa sechs Stunden, mindestens aber bis alle Plättchen visuell betrachtet trocken sind. Die Qualität der trockenen Prüfplättchen wurde überprüft. Plättchen mit Luftblasen auf der Anschmutzung oder die Unregelmäßigkeiten aufweisen wurden aussortiert. Auf alle anderen Plättchen wurde je ein oranger Markierungspunkt geklebt. Bis zur Verwendung im Tauchversuch wurden die Prüfplättchen in Reagenzgläsern mit Schraubverschluss bei Raumtemperatur gelagert.

### Durchführung Tauchversuch:

| | |
|---|---|
| Konzentration: | 2 ml/l |
| Wasserqualitäten: | VE-Wasser |
| Temperatur: | 45°C ± 1°C |
| Haltezeit: | 4 und 10 min |
| Rührgeschwindigkeit: | 350 U/min (Rührer IKA RCT classic) |
| Vorlage: | 1000 ml Lösung im 1000ml-Becherglas |
| Prüfplättchen: | Heparinisiertes, reakt. Schafblut |
| | Iod-Blut |

Die angeschmutzten Prüfplättchen wurden jeweils einzeln in die Lösung getaucht. Nach dem Herausnehmen erfolgte ein kurzes Nachtauchen in kaltem VE-Wasser. Die Plättchen trockneten liegend bei Raumtemperatur. Die visuelle Auswertung erfolgte mit den getrockneten Plättchen. Die Prüfplättchen mit der Anschmutzung heparinisiertes, reaktiviertes Schafblut wurden mit einer 0,1%igen Amidoschwarzlösung angefärbt.

### c. Auswertung

Die Auswertung erfolgte visuell mit den getrockneten Plättchen. Zusätzlich erfolgte auch hier die Auswertung mithilfe der Integrale der verbleibenden Blutrückstände in Relation zur Gesamtfläche des Prüfkörpers unter Zuhilfenahme der Software ImageJ.

### d. Ergebnisse

Die folgende Tabelle 1 fasst die Versuchsergebnisse zusammen. Neben den in der Tabelle 1 aufgelisteten Bestandteilen wurden die getesteten flüssigen Reinigungsmittelkonzentrate ferner aus den folgenden, ansonsten gleichbleibenden Bestandteilen hergestellt:

| | |
|---|---|
| 3,5 Gew.-% | Endoprotease |
| 8,0 Gew.-% | 45%ige KOH |
| 16 Gew.-% | 99%iges Triethanolamin |
| 12,0 Gew.-% | 40%ige MGDA, 3Na |
| 10 Gew.-% | 50%ige PBTC |
| auf 100 Gew.-% | Wasser |

**Tabelle 1: Versuchsergebnisse zum Pumpendruck und zur Reinigungsleistung.**

| | **Fettalkoholalkoxylat** | **Aminosäurebasiertes Tensid** | **Hydrotrop** | **Pumpendruck** | **Reinigungsleistung** |
|---|---|---|---|---|---|
| **1** | 0,5 Gew.-% FA C12/C14 2EO/6PO | 0,5 Gew.-% 30%iges Na-Lauroylsarcosinat | 0,8 Gew.-% 42%iges Na-Octylsulfat | sehr gut | sehr gut |
| **2** | 0,5 Gew.-% FA C12/C14 2EO/6PO | 0,5 Gew.-% 30%iges Na-Lauroylsarcosinat | 1,6 Gew.-% 42%iges Na-Ethylhexylsulfat | Gut | sehr gut |
| **3** | 0,5 Gew.-% FA C12/C14 2EO/6PO | - | 1,7 Gew.-% 42%iges Na-Octylsulfat | sehr gut | mittelmäßig |
| **4** | 0,5 Gew.-% FA C12/C14 2EO/6PO | 0,8 Gew.-% 30%iges Na-Lauroylsarcosinat | - | sehr schlecht | sehr schlecht |
| **5** | 0,5 Gew.-% FA C12/C14 2EO/6PO | - | 15 Gew.-% 40%i-ges Na-Cumolsulfonat | sehr gut | schlecht |

Das flüssige Reinigungsmittelkonzentrat umfassend eine Kombination aus Fettalkoholalkoxylat und aminosäurebasiertem Tensid ohne Zugabe eines Hydrotrops (Vergleichsversuch 4) zeigt die schlechteste Reinigungsleistung und das schlechteste Pumpendruckverhalten der Versuchsreihe. Das zweitschlechteste Reinigungsergebnis erzielt das flüssige Reinigungsmittelkonzentrat mit Natrium-Cumolsulfonat (Vergleichsversuch 5), wobei hier ein sehr gutes Druckverhalten beobachtet wird. Das flüssige Reinigungsmittelkonzentrat umfassend Fettalkoholalkoxylat und das Hydrotrop Natrium-Octylsulfat (Vergleichsversuch 3) zeigt ein sehr gutes Pumpendruckverhalten bei nur mittelmäßiger Reinigungsleistung. Der Zusatz des in alleiniger Kombination mit dem Fettalkoholalkoxylat schlecht reinigenden aminosäurebasierten Tensids (Vergleichsversuch 4) führte zu einer sehr guten Reinigungsleistung des flüssigen Reinigungsmittelkonzentrats bei einem guten Pumpendruckverlauf im maschinellen Versuch (Versuch 2).

Die erfindungsgemäßen flüssigen Reinigungsmittelkonzentrate mit den Kombinationen von Fettalkoholalkoxylat, aminosäurebasiertem Tensid und Hydrotrop (Versuche 1 und 2) erzielen die besten Reinigungsleistungen verglichen mit den jeweiligen Einzelkomponenten und den verschiedenen Kombinationen zweier Bestandteile (Vergleichsversuche 3 bis 5). Es wird ein synergistischer Effekt der erfindungsgemäßen Kombination von Fettalkoholalkoxylat, aminosäurebasiertem Tensid und Hydrotrop hinsichtlich der Entfernung von Blutrückständen beobachtet. Die Reinigungsleistung der Dreierkombination in den durchgeführten Versuchen ist stets besser als die der Summe der Einzelkomponenten.

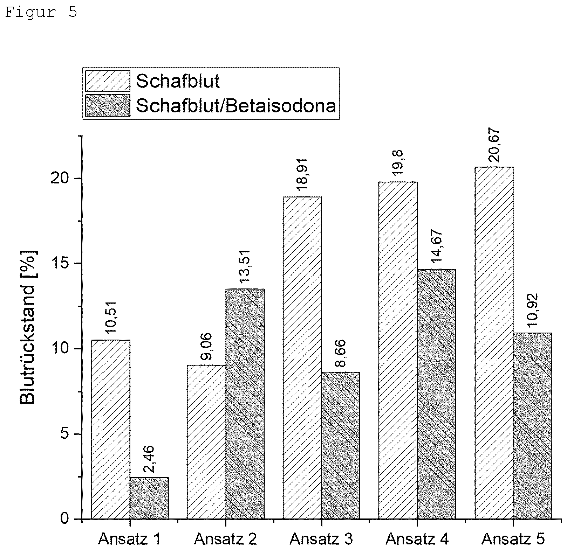

Figur 5 zeigt eine quantitative Bestimmung der Blutrückstände nach Durchführung der Tauchversuche mit Schafsblut sowie einer Mischung aus Schafsblut und Betaisodona-Tinktur. Gezeigt sind die Ergebnisse für die in der Tabelle 1 aufgeführten Ansätze 1 bis 5.

Ein weiteres Ausführungsbeispiel für ein erfindungsgemäßes Reinigungsmittelkonzentrat, welches annährend die die gleichen Versuchsergebnisse erzielt, wie das flüssige Reinigungsmittelkonzentrat aus Versuchsbeispiel 1, besteht aus den folgenden Bestandteilen:

| | |
|---|---|
| 0,5 Gew.-% | FA C12/C15 2EO/6PO |
| 0,1 Gew.-% | 96%iges Cocoyl-/Lauroylglutamat |
| 0,7 Gew.-% | 42%iges Na-Octylsulfat |
| 1 Gew.-% | Endoprotease |
| 14 Gew.-% | 99%iges Monoethanolamin |
| 6 Gew.-% | Aminocarboxylat |
| 1 Gew.-% | 75%ige Phosphorsäure |
| auf 100 Gew.-% | Wasser |

### 2. Kaltwasserdosierung

Im Stand der Technik erfolgt das Eindosieren von tensidhaltigen, enzymatischen, mildalkalischen, flüssigen Reinigungsmitteln für die maschinelle Reinigung üblicherweise bei einer Wassertemperatur von etwa 40°C. Dieses ist notwendig, da bei geringeren Temperaturen eine zu starke Schaumneigung der Reinigungsmittel vorliegt. Nachteilig an der Dosierung bei einer Temperatur von etwa 40°C ist allerdings, dass die Laufzeit der Reinigungsprogramme verlängert wird, da zunächst eine gewisse Dauer für das Aufheizen von der Einlauftemperatur (meist ca. 18-22°C) bis auf eine Temperatur von 40°C abgewartet werden muss, bevor das Reinigungsmittel wirken kann.

Das erfindungsgemäße flüssige Reinigungsmittelkonzentrat sowie die gebrauchsfertige Anwendungslösung ermöglichen hingegen eine Kaltdosierung bereits direkt nach dem Wassereinlauf, vorzugsweise bei einer Temperatur von 38°C oder weniger, noch weiter vorzugsweise von 18 bis 35°C, noch weiter vorzugsweise von 20 bis 30°C, noch weiter vorzugsweise von 22 bis 27°C, noch weiter vorzugsweise bei etwa 25°C, ohne dass es zu einem Programmabbruch aufgrund von zu starker Schaumentwicklung kommt. Dies ist mit den aus dem Stand der Technik bekannten Reinigungsmitteln derzeit nicht möglich.

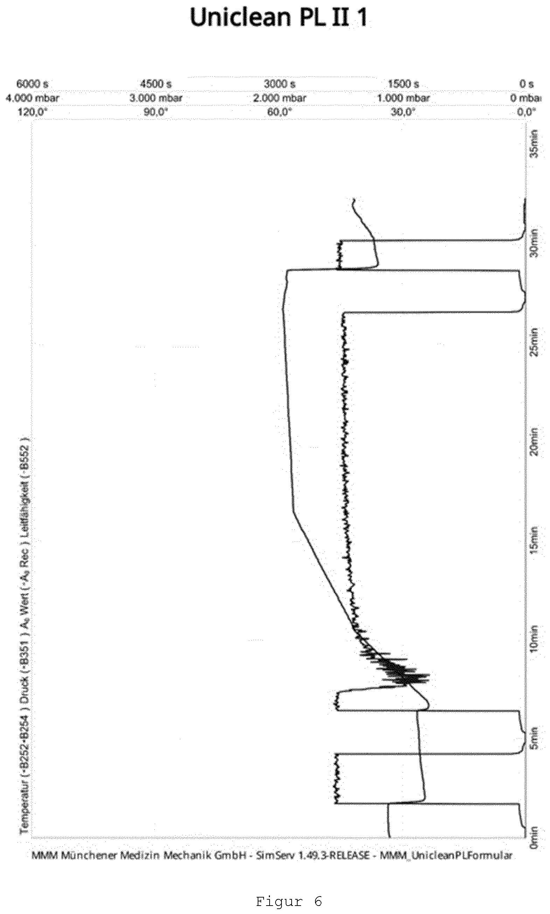

In Figur 6 wird der korrekte und vollständige Programmablauf (Druck- und Temperaturkurven) aus einer Reinigungs- und Desinfektionsanlage (UniClean PL II der Firma MMM) dargestellt. Hierbei erfolgte die Dosierung des erfindungsgemäßen flüssigen Reinigungsmittelkonzentrats mit einer Konzentration von 3 ml/l bei 25°C.

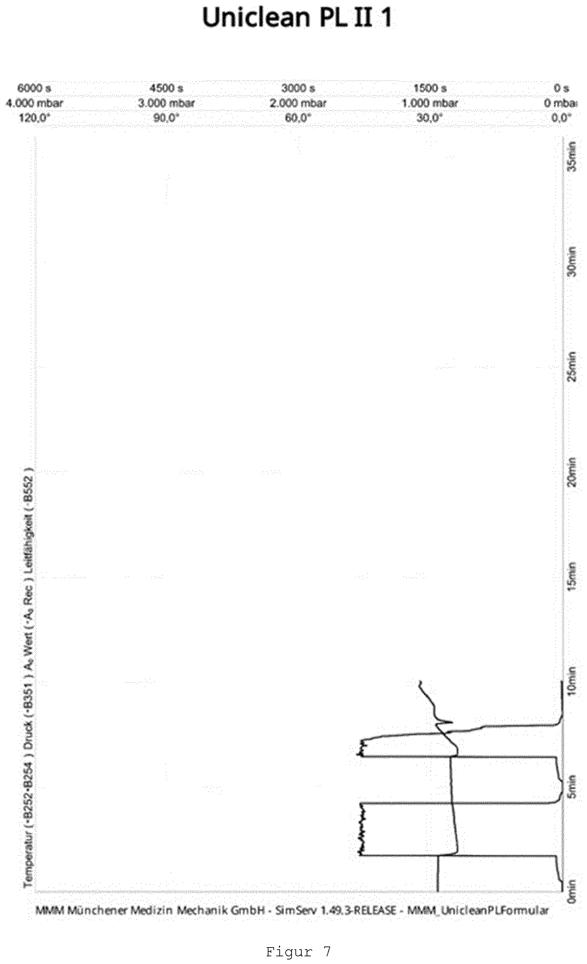

Analog wurde dieses für einige der aus dem Stand der Technik bekannten und marktüblichen Reiniger mit der jeweils standardmäßig empfohlenen Konzentration bei 25°C durchgeführt (u.a. Dr. Weigert, neodisher MediClean forte, 6 ml/l, #681964; Ruhof, Endozyme AW plus, 3,5 ml/l; Dr. Schumacher, thermoshield Xtreme, 3 ml/l, #460764; Borer, deconex Twin pH10 + Twin Zyme, 3 ml/l + 1,5 ml/l, #0370073 + #0,397577; Prolystica, Prolystica 2x concentrate alkaline cleaner, 3 ml/l, #290186). Für dieser Reinigungsmittel wird ein Programmabbruch, wie er in Figur 7 dargestellt ist, beobachtet.

## Patentansprüche

1. Flüssiges Reinigungsmittelkonzentrat zur maschinellen Reinigung und/oder Desinfektion von medizinischen und/oder chirurgischen Instrumenten und/oder Apparaten, umfassend:
a. mindestens ein Fettalkoholalkoxylat,
b. mindestens ein aminosäurebasiertes Tensid,
c. mindestens ein Hydrotrop, und
d. mindestens ein Enzym, vorzugsweise proteolytisches Enzym,
wobei ein pH-Wert des flüssigen Reinigungsmittelkonzentrats 9 oder >9 ist.

2. Flüssiges Reinigungsmittelkonzentrat nach Anspruch 1, **dadurch gekennzeichnet, dass** das flüssige Reinigungsmittelkonzentrat einen pH-Wert 9-12, vorzugsweise 10-12, weiter vorzugsweise 10-11, aufweist.

3. Flüssiges Reinigungsmittelkonzentrat nach Anspruch 1 oder 2, **dadurch gekennzeichnet, dass** das mindestens eine Fettalkoholalkoxylat ausgewählt ist aus Fettalkoholethoxylaten (FAEO), Fettalkoholpropoxylaten (FAPO), butylveretherten Fettalkoholethoxylaten (FAEOBV), butylveretherten Fettalkoholpropoxylaten (FAPOBV), methylveretherten Fettalkoholethoxylaten (FAEOMV), methylveretherten Fettalkoholpropoxylaten (FAPOMV), fettalkoholbasierten EO/PO-Copolymeren (FAEOPO), butylveretherten fettalkoholbasierten EO/PO-Copolymeren (FAEOPOBV) und methylveretherten fettalkoholbasierten EO/PO-Copolymeren (FAEOPOMV), wobei das Fettalkoholalkoxylat vorzugsweise ein fettalkoholbasiertes EO/PO-Copolymer ist.

4. Flüssiges Reinigungsmittelkonzentrat nach einem der Ansprüchen 1 bis 3, **dadurch gekennzeichnet, dass** das Fettalkoholalkoxylat mindestens eines der folgenden Merkmale aufweist:
- 0-10 EO-Einheiten, vorzugsweise 1-4 EO-Einheiten, weiter vorzugsweise 1-2 EO-Einheiten umfasst,
- 0-8 PO-Einheiten, vorzugsweise 1-8 PO-Einheiten, weiter vorzugsweise 4-8 PO-Einheiten umfasst,
- mindestens einen C6-C16-Fettalkoholrest, vorzugsweise C12-C15-Fettalkoholrest aufweist,
- ausgewählt ist aus der Gruppe bestehend aus C12-C15-Fettakoholrest mit 2EO/6PO-Einheiten, C12-C15-Fettal-koholrest mit 8EO/4PO-Einheiten, butyl- oder methylverethertem C12-C14-Fettalkoholrest mit 10EO-Einheiten, C10-C12-Fettalkoholrest mit 6EO/8PO-Einheiten, C12-C14-Fettalkoholrest mit 2EO/4PO-Einheiten, C12-C14-Fettalkoholrest mit 4EO/5PO-Einheiten, methylverethertem C13-C15-Fettalkoholrest mit 5EO/3PO-Einheiten und C13-C15-Fettalkoholrest mit 5EO/3PO-Einheiten, vorzugsweise ausgewählt ist aus C12-C15-Fettakoholrest mit 2EO/6PO-Einheiten, C12-C14-Fettalkoholrest mit 2EO/4PO-Einheiten und C12-C14-Fettalkoholrest mit 4EO/5PO-Einheiten.

5. Flüssiges Reinigungsmittelkonzentrat nach einem der Ansprüche 1 bis 4, **dadurch gekennzeichnet, dass** das aminosäurebasierte Tensid mindestens eines der folgenden Merkmale aufweist:
- das aminosäurebasierte Tensid ausgewählt ist aus Verbindungen mit einem gesättigten oder einfach ungesättigten C10-C18-Kohlenstoffrest, vorzugsweise einem gesättigten C12-C16-Kohlenstoffrest;
- das aminosäurebasierte Tensid ausgewählt ist aus Sarcosinen, Taurinen, Glutaminsäuren und deren Salzen, vorzugsweise Sarcosinen und deren Natriumsalzen;
- das aminosäurebasierte Tensid ausgewählt ist aus Lauroylsarcosin, Oleoylsarcosin, Myristoylsarcosin, Stearoylsarcosin und Lauroylglutaminsäure und deren Salzen, vorzugsweise Lauroylsarcosin und Lauroylglutaminsäure und deren Natriumsalzen.

6. Flüssiges Reinigungsmittelkonzentrat nach einem der Ansprüche 1 bis 5, **dadurch gekennzeichnet, dass** das mindestens eine Hydrotrop ausgewählt ist aus:
- Alkylsulfaten, vorzugsweise C6-C10-Alkylsulfaten und deren Natriumsalze, weiter vorzugsweise Natrium-Octylsulfat und Natrium-Ethylhexylsulfat;
- Alkylsulfonaten, vorzugsweise C6-C10-Alkylsulfonaten;
- aromatischen Sulfonaten, vorzugsweise Xylolsulfonat, *p*-Toluolsulfonat und deren Natriumsalze;
- Propionaten, vorzugsweise iso-Octyliminodipropionat, *n*-Octyliminodipropionat, Capryl- und Caprinamphopropionat;
- C4-C10-Alkylethercarbonsäuren mit 4-10 EO-Einheiten, vorzugsweise Alkyl(8)polyethercarbonsäure mit 8 EO-Einheiten und Alkyl(4-8)polyethercarbonsäure mit 5 EO-Einheiten;
- Alkylglycosiden, Alkyldiglycosiden, Alkylpolyglycosiden und Gemischen hiervon, wobei der Alkylrest vorzugsweise ein verzweigter oder unverzweigter C4-C16-Alkylrest ist und der Glycosidrest vorzugsweise ausgewählt ist aus Hexoseeinheit und Pentoseeinheit, weiter vorzugsweise ausgewählt ist aus Glucopyranoseeinheit und Xylopyranoseeinheit.

7. Flüssiges Reinigungsmittelkonzentrat nach einem der Ansprüche 1 bis 5, **dadurch gekennzeichnet, dass** das Hydrotrop Natrium-Octylsulfat, Natrium-Ethylhexylsulfat oder ein Gemisch hiervon ist.

8. Flüssiges Reinigungsmittelkonzentrat nach einem der Ansprüche 1 bis 7, **dadurch gekennzeichnet, dass** das flüssige Reinigungsmittelkonzentrat ferner weitere Bestandteile ausgewählt aus Alkanolaminen, Alkalimetallhydroxiden, Komplexbildern, Lösungsmitteln, Korrosionsschutzmitteln, Duftstoffen und Farbstoffen umfasst.

9. Flüssiges Reinigungsmittelkonzentrat nach Anspruch 8, **dadurch gekennzeichnet, dass** das Alkalimetallhydroxid Kaliumhydroxid ist.

10. Flüssiges Reinigungsmittelkonzentrat nach einem der Ansprüche 1 bis 9, **dadurch gekennzeichnet, dass** mindestens einer der folgenden Bestandteile in dem flüssigen Reinigungsmittelkonzentrat jeweils in den folgenden Gewichtsanteilen enthalten ist:
- das Fettalkoholalkoxylat in einem Gewichtsanteil von 0,1 bis 9 Gew.-%, vorzugsweise von 0,4 bis 2 Gew.-%, bezogen auf die Gesamtmasse des flüssigen Reinigungsmittelkonzentrats;
- das aminosäurebasierte Tensid in einem Gewichtsanteil von 0,05 bis 5 Gew.-%, vorzugsweise 0,1 bis 2 Gew.-%, bezogen auf die Gesamtmasse des flüssigen Reinigungsmittelkonzentrats;
- das Hydrotrop in einem Gewichtsanteil von 0,05 bis 13 Gew.-%, vorzugsweise von 0,1 bis 7 Gew.-%, weiter vorzugsweise von 0,15 bis 3,5 Gew.-% bezogen auf die Gesamtmasse des flüssigen Reinigungsmittelkonzentrat;
- das Enzym in einem Gewichtsanteil von 0,05 bis 4 Gew.-%, vorzugsweise von 0,1 bis 2 Gew.-%, bezogen auf die Gesamtmasse des flüssigen Reinigungsmittelkonzentrats.

11. Gebrauchsfertige Anwendungslösung umfassend 0,05 bis 99,9% des flüssigen Reinigungsmittelkonzentrats nach einem der Ansprüche 1 bis 10, wobei ein pH-Wert der gebrauchsfertigen Anwendungslösung 9 oder >9 ist.

12. Gebrauchsfertige Anwendungslösung nach Anspruch 11, **dadurch gekennzeichnet, dass** das flüssige Reinigungsmittelkonzentrat einen pH-Wert 9-12, vorzugsweise 10-12, weiter vorzugsweise 10-11, aufweist.

13. Verwendung des flüssigen Reinigungsmittelkonzentrats nach einem der Ansprüche 1 bis 10 oder der gebrauchsfertigen Anwendungslösung nach einem der Ansprüche 11 oder 12 zur Reinigung und/oder Desinfektion von Gegenständen, vorzugsweise zur maschinellen Reinigung und/oder Desinfektion von Gegenständen.

14. Verwendung nach Anspruch 13, **dadurch gekennzeichnet, dass** es sich bei den Gegenständen um medizinische und/oder chirurgische Instrumente und/oder Apparate handelt.

15. Verwendung nach Anspruch 13 oder 14, **dadurch gekennzeichnet, dass** das flüssige Reinigungsmittelkonzentrat oder die gebrauchsfertige Anwendungslösung kalt dosiert werden, vorzugsweise bei einer Temperatur von 38°C oder weniger, weiter vorzugsweise von 18 bis 35°C, noch weiter vorzugsweise von 20 bis 30°C, noch weiter vorzugsweise von 22 bis 27°C, noch weiter vorzugsweise bei etwa 25°C.

16. Verfahren zur Reinigung von medizinischen und/oder chirurgischen Instrumenten und/oder Apparaten, **gekennzeichnet durch** folgende Schritte:
a) Herstellen einer gebrauchsfertigen Anwendungslösung nach einem der Ansprüche 11 oder 12,
b) Reinigen der medizinischen und/oder chirurgischen Instrumente und/oder Apparate mit der gebrauchsfertigen Anwendungslösung.

17. Verfahren nach Anspruch 16, **dadurch gekennzeichnet, dass** die gebrauchsfertige Anwendungslösung kalt hergestellt wird, vorzugsweise bei einer Temperatur von 38°C oder weniger, weiter vorzugsweise von 18 bis 35°C, noch weiter vorzugsweise von 20 bis 30°C, noch weiter vorzugsweise von 22 bis 27°C, noch weiter vorzugsweise bei etwa 25°C.

## Claims

1. Liquid cleaning agent concentrate for machine cleaning and/or disinfection of medical and/or surgical instruments and/or apparatus, comprising:
a. at least one fatty alcohol alkoxylate,
b. at least one amino acid-based surfactant,
c. at least one hydrotrope, and
d. at least one enzyme, preferably proteolytic enzyme,
wherein the pH of the liquid cleaning agent concentrate is 9 or >9.

2. Liquid cleaning agent concentrate according to Claim 1, **characterized in that** the liquid cleaning agent concentrate has a pH of 9-12, preferably 10-12, more preferably 10-11.

3. Liquid cleaning agent concentrate according to Claim 1 or 2, **characterized in that** the at least one fatty alcohol alkoxylate is selected from fatty alcohol ethoxylates (FAEO), fatty alcohol propoxylates (FAPO), butyl-etherified fatty alcohol ethoxylates (FAEOBV), butyl-etherified fatty alcohol propoxylates (FAPOBV), methyl-etherified fatty alcohol ethoxylates (FAEOMV), methyl-etherified fatty alcohol propoxylates (FAPOMV), fatty alcohol-based EO/PO copolymers (FAEOPO), butyl-etherified fatty alcohol-based EO/PO copolymers (FAEOPOBV) and methyl-etherified fatty alcohol-based EO/PO copolymers (FAEOPOMV), wherein the fatty alcohol alkoxylate is preferably a fatty alcohol-based EO/PO copolymer.

4. Liquid cleaning agent concentrate according to any of Claims 1 to 3, **characterized in that** the fatty alcohol alkoxylate has at least one of the following features:
- comprises 0-10 EO units, preferably 1-4 EO units, more preferably 1-2 EO units,
- comprises 0-8 PO units, preferably 1-8 PO units, more preferably 4-8 PO units,
- has at least one C6-C16 fatty alcohol radical, preferably C12-C15 fatty alcohol radical,
- is selected from the group consisting of C12-C15 fatty alcohol radical having 2 EO/6 PO units, C12-C15 fatty alcohol radical having 8 EO/4 PO units, butyl- or methyl-etherified C12-C14 fatty alcohol radical having 10 EO units, C10-C12 fatty alcohol radical having 6 EO/8 PO units, C12-C14 fatty alcohol radical having 2 EO/4 PO units, C12-C14 fatty alcohol radical having 4 EO/5 PO units, methyl-etherified C13-C15 fatty alcohol radical having 5 EO/3 PO units and C13-C15 fatty alcohol radical having 5 EO/3 PO units, preferably selected from C12-C15 fatty alcohol radical having 2 EO/6 PO units, C12-C14 fatty alcohol radical having 2 EO/4 PO units and C12-C14 fatty alcohol radical having 4 EO/5 PO units.

5. Liquid cleaning agent concentrate according to any of Claims 1 to 4, **characterized in that** the amino acid-based surfactant has at least one of the following features:
- the amino acid-based surfactant is selected from compounds having a saturated or monounsaturated C10-C18 carbon radical, preferably a saturated C12-C16 carbon radical;
- the amino acid-based surfactant is selected from sarcosines, taurines, glutamic acids and salts thereof, preferably sarcosines and sodium salts thereof;
- the amino acid-based surfactant is selected from lauroyl sarcosine, oleoyl sarcosine, myristoyl sarcosine, stearoyl sarcosine and lauroyl glutamic acid and salts thereof, preferably lauroyl sarcosine and lauroyl glutamic acid and sodium salts thereof.

6. Liquid cleaning agent concentrate according to any of Claims 1 to 5, **characterized in that** the at least one hydrotrope is selected from:
- alkyl sulfates, preferably C6-C10-alkyl sulfates and sodium salts thereof, more preferably sodium octyl sulfate and sodium ethylhexyl sulfate;
- alkyl sulfonates, preferably C6-C10-alkyl sulfonates;
- aromatic sulfonates, preferably xylene sulfonate, p-toluenesulfonate and sodium salts thereof;
- propionates, preferably isooctylimino dipropionate, n-octylimino dipropionate, caprylic and capric amphopropionate;
- C4-C10-alkyl ether carboxylic acids having 4-10 EO units, preferably alkyl(8) polyether carboxylic acid having 8 EO units and alkyl(4-8) polyether carboxylic acid having 5 EO units;
- alkyl glycosides, alkyl diglycosides, alkyl polyglycosides and mixtures thereof, wherein the alkyl radical is preferably a branched or unbranched C4-C16-alkyl radical and the glycoside radical is preferably selected from hexose unit and pentose unit, more preferably selected from glucopyranose unit and xylopyranose unit.

7. Liquid cleaning agent concentrate according to any of Claims 1 to 5, **characterized in that** the hydrotrope is sodium octyl sulfate, sodium ethylhexyl sulfate or a mixture thereof.

8. Liquid cleaning agent concentrate according to any of Claims 1 to 7, **characterized in that** the liquid cleaning agent concentrate also comprises further constituents selected from alkanolamines, alkali metal hydroxides, chelating agents, solvents, corrosion inhibitors, fragrances and dyes.

9. Liquid cleaning agent concentrate according to Claim 8, **characterized in that** the alkali metal hydroxide is potassium hydroxide.

10. Liquid cleaning agent concentrate according to any of Claims 1 to 9, **characterized in that** at least one of the following constituents in the liquid cleaning agent concentrate is in each case present at the following proportions by weight:
- the fatty alcohol alkoxylate at a proportion by weight of 0.1 to 9% by weight, preferably from 0.4 to 2% by weight, based on the total mass of the liquid cleaning agent concentrate;
- the amino acid-based surfactant at a proportion by weight of 0.05 to 5% by weight, preferably 0.1 to 2% by weight, based on the total mass of the liquid cleaning agent concentrate;
- the hydrotrope at a proportion by weight of 0.05 to 13% by weight, preferably from 0.1 to 7% by weight, more preferably from 0.15 to 3.5% by weight, based on the total mass of the liquid cleaning agent concentrate;
- the enzyme at a proportion by weight of 0.05 to 4% by weight, preferably from 0.1 to 2% by weight, based on the total mass of the liquid cleaning agent concentrate.

11. Ready-to-use application solution comprising 0.05 to 99.9% of the liquid cleaning agent concentrate according to any of Claims 1 to 10, wherein the pH of the ready-to-use application solution is 9 or >9.

12. Ready-to-use application solution according to Claim 11, **characterized in that** the liquid cleaning agent concentrate has a pH of 9-12, preferably 10-12, more preferably 10-11.

13. Use of the liquid cleaning agent concentrate according to any of Claims 1 to 10 or of the ready-to-use application solution according to either of Claims 11 or 12 for cleaning and/or disinfection of objects, preferably for machine cleaning and/or disinfection of objects.

14. Use according to Claim 13, **characterized in that** the objects are medical and/or surgical instruments and/or apparatus.

15. Use according to Claim 13 or 14, **characterized in that** the liquid cleaning agent concentrate or the ready-to-use application solution is dispensed cold, preferably at a temperature of 38°C or less, more preferably from 18 to 35°C, even more preferably from 20 to 30°C, still more preferably from 22 to 27°C, even further preferably at about 25°C.

16. Method for cleaning medical and/or surgical instruments and/or apparatus, **characterized by** the following steps:
a) preparing a ready-to-use application solution according to either of Claims 11 or 12,
b) cleaning the medical and/or surgical instruments and/or apparatus with the ready-to-use application solution.

17. Method according to Claim 16, **characterized in that** the ready-to-use application solution is prepared cold, preferably at a temperature of 38°C or less, more preferably from 18 to 35°C, even more preferably from 20 to 30°C, still more preferably from 22 to 27°C, even further preferably at about 25°C.

## Revendications

1. Détergent liquide concentré pour le nettoyage mécanique et/ou la désinfection d'instruments et/ou d'appareils médicaux et/ou chirurgicaux, comprenant :
a. au moins un alcool gras alcoxylé,
b. au moins un tensioactif à base d'acides aminés,
c. au moins un hydrotrope, et
d. au moins une enzyme, de préférence une enzyme protéolytique,
le pH du détergent liquide concentré étant égal à 9 ou > 9.

2. Détergent liquide concentré selon la revendication 1, **caractérisé en ce que** le détergent liquide concentré présente un pH de 9-12, de préférence de 10-12, plus particulièrement de 10-11.

3. Détergent liquide concentré selon la revendication 1 ou 2, **caractérisé en ce que** l'au moins un alcool gras alcoxylé est choisi parmi les alcools gras éthoxylés (FAEO), les alcools gras propoxylés (FAPO), les alcools gras éthoxylés éthérifiés avec des groupes butyle (FAEOBV), les alcools gras propoxylés éthérifiés avec des groupes butyle (FAPOBV), les alcools gras éthoxylés éthérifiés avec des groupes méthyle (FAEOMV), les alcools gras propoxylés éthérifiés avec des groupes méthyle (FAPOMV), les copolymères OE/OP à base d'alcools gras (FAEOPO), les copolymères OE/OP à base d'alcools gras éthérifiés avec des groupes butyle (FAEOPOBV) et les copolymères OE/OP à base d'alcools gras éthérifiés avec des groupes méthyle (FAEOPOMV), l'alcool gras alcoxylé étant de préférence un copolymère OE/OP à base d'alcools gras.

4. Détergent liquide concentré selon l'une des revendications 1 à 3, **caractérisé en ce que** l'alcool gras alcoxylé présente au moins l'une des caractéristiques suivantes :
- il comprend 0-10 motifs OE, de préférence 1-4 motifs OE, plus particulièrement 1-2 motifs OE,
- il comprend 0-8 motifs OP, de préférence 1-8 motifs PO, plus particulièrement 4-8 motifs PO,
- il comprend au moins un radical alcool gras en C6-C16, de préférence un radical alcool gras en C12-C15,
- il est choisi dans le groupe consistant en un radical alcool gras en C12-C15 à 2 motifs OE/6 motifs OP, un radical alcool gras en C12-C15 à 8 motifs OE/4 motifs OP, un radical alcool gras en C12-C14 éthérifié avec des groupes butyle ou méthyle à 10 motifs OE, un radical alcool gras en C10-C12 à 6 motifs OE/8 motifs OP, un radical alcool gras en C12-C14 à 2 motifs OE/4 motifs OP, un radical alcool gras en C12-C14 à 4 motifs OE/5 motifs OP, un radical alcool gras en C13-C15 éthérifié par des groupes méthyle à 5 motifs OE/3 motifs OP et un radical alcool gras en C13-C15 à 5 motifs OE/3 motifs OP, de préférence choisi parmi un radical alcool gras en C12-C15 à 2 motifs OE/6 motifs OP, un radical alcool gras en C12-C14 à 2 motifs OE/4 motifs OP et un radical alcool gras en C12-C14 à 4 motifs OE/5 motifs OP.

5. Détergent liquide concentré selon l'une des revendications 1 à 4, **caractérisé en ce que** le tensioactif à base d'acides aminés présente au moins l'une des caractéristiques suivantes :
- le tensioactif à base d'acides aminés est choisi parmi les composés ayant un radical carboné en C10-C18 saturé ou monoinsaturé, de préférence un radical carboné en C12-C16 saturé ;
- le tensioactif à base d'acides aminés est choisi parmi les sarcosines, les taurines, les acides glutamiques et leurs sels, de préférence les sarcosines et leurs sels de sodium ;
- le tensioactif à base d'acides aminés est choisi parmi la lauroylsarcosine, l'oléoylsarcosine, la myristoylsarcosine, la stéaroylsarcosine et l'acide lauroylglutamique et leurs sels, de préférence la lauroylsarcosine et l'acide lauroylglutamique et leurs sels.

6. Détergent liquide concentré selon l'une des revendications 1 à 5, **caractérisé en ce que** l'au moins un hydrotrope est choisi parmi :
- les alkylsulfates, de préférence les alkylsulfates en C6-C10 et leurs sels de sodium, plus particulièrement l'octylsulfate de sodium et l'éthylhexylsulfate de sodium ;
- les alkylsulfonates, de préférence les alkylsulfonates en C6-C10 ;
- les sulfonates aromatiques, de préférence le xylènesulfonate, le p-toluènesulfonate et leurs sels de sodium ;
- les propionates, de préférence l'iminodipropionate d'iso-octyle, l'iminodipropionate de n-octyle, le caprylamphopropionate et l'amphopropionate de l'acide caprique ;
- les acides (alkyle en C4-C10) carboxyliques éthoxylés à 4-10 motifs OE, de préférence les acides alkylcarboxyliques 8-polyéthoxylés à 8 motifs OE et un acide alkylcarboxylique 4-8 polyéthoxylé à 5 motifs OE ;
- les alkylglycosides, les alkyldiglycosides, les alkylpolyglycosides et les mélanges de ceux-ci, le radical alkyle étant de préférence un radical alkyle en C4-C16 ramifié ou non ramifié et le radical glycoside étant de préférence choisi parmi un motif hexose et un motif pentose, plus particulièrement est choisi parmi un motif glucopyranose et un motif xylopyranose.

7. Détergent liquide concentré selon l'une des revendications 1 à 5, **caractérisé en ce que** l'hydrotrope est l'octylsulfate de sodium, l'éthylhexylsulfate de sodium ou un mélange de ceux-ci.

8. Détergent liquide concentré selon l'une des revendications 1 à 7, **caractérisé en ce que** le détergent liquide concentré comprend en outre d'autres constituants choisis parmi les alcanolamines, les hydroxydes de métaux alcalins, les complexants, les solvants, les agents anticorrosion, les parfums et les colorants.

9. Détergent liquide concentré selon la revendication 8, **caractérisé en ce que** l'hydroxyde d'un métal alcalin est l'hydroxyde de potassium.

10. Détergent liquide concentré selon l'une des revendications 1 à 9, **caractérisé en ce qu'**il contient au moins l'un des constituants suivants dans le détergent liquide concentré, chacun en les pourcentages en poids suivants :
- l'alcool gras alcoxylé selon une proportion en poids de 0,1 à 9 % en poids, de préférence de 0,4 à 2 % en poids, par rapport à la masse totale du détergent liquide concentré ;
- le tensioactif à base d'acides aminés selon une proportion en poids de 0,05 à 5 % en poids, de préférence de 0,1 à 2 % en poids, par rapport à la masse totale du détergent liquide concentré ;
- l'hydrotrope selon une proportion en poids de 0,05 à 13 % en poids, de préférence de 0,1 à 7 % en poids, plus particulièrement de 0,15 à 3,5 % en poids, par rapport à la masse totale du détergent liquide concentré ;
- l'enzyme selon une proportion pondérale de 0,05 à 4 % en poids, de préférence de 0,1 à 2 % en poids, par rapport à la masse totale du détergent liquide concentré.

11. Solution d'utilisation prête à l'emploi comprenant 0,05 à 99,9 % du détergent liquide concentré selon l'une des revendications 1 à 10, le pH de la solution d'utilisation prête à l'emploi étant égal à 9 ou > 9.

12. Solution d'utilisation prête à l'emploi selon la revendication 11, **caractérisée en ce que** le détergent liquide concentré présente un pH de 9-12, de préférence de 10-12, plus particulièrement de 10-11.

13. Utilisation du détergent liquide concentré selon l'une des revendications 1 à 10 ou de la solution d'utilisation prête à l'emploi selon l'une des revendications 11 ou 12 pour le nettoyage et/ou la désinfection d'objets, de préférence pour le nettoyage et/ou la désinfection mécanique d'objets.

14. Utilisation selon la revendication 13, **caractérisée en ce que** les objets sont des instruments et/ou des appareils médicaux et/ou chirurgicaux.

15. Utilisation selon la revendication 13 ou 14, **caractérisée en ce que** le détergent liquide concentré ou la solution d'utilisation prête à l'emploi est dosé à froid, de préférence à une température de 38 °C ou moins, plus particulièrement de 18 à 35 °C, plus spécialement de 20 à 30 °C, plus spécialement de 22 à 27 °C, plus spécialement à environ 25 °C.

16. Procédé de nettoyage d'instruments et/ou d'appareils médicaux et/ou chirurgicaux, **caractérisé par** les étapes suivantes :
a) fabrication d'une solution d'utilisation prête à l'emploi selon l'une des revendications 11 ou 12,
b) nettoyage des instruments et/ou des appareils médicaux et/ou chirurgicaux avec la solution d'utilisation prête à l'emploi.

17. Procédé selon la revendication 16, **caractérisé en ce que** la solution d'utilisation prête à l'emploi est fabriquée à froid, de préférence à une température de 38 °C ou moins, plus particulièrement de 18 à 35 °C, plus spécialement de 20 à 30 °C, plus spécialement de 22 à 27 °C, plus spécialement à environ 25 °C.
